# NEW EUROPEAN PATENT SPECIFICATION

(11) **EP 0 757 550 B2**
(45) Date of publication and mention of the opposition decision: **15.09.2004**
(45) Mention of the grant of the patent: 09.12.1998
(21) Application number: 95918878.0
(22) Date of filing: 28.04.1995
(51) Int. Cl.: A61F 13/56, A61F 13/15

(54) **CLOSURE SYSTEM FOR DISPOSABLE PULL-ON PANTS HAVING A STRETCHABLE WAISTBAND**
VERSCHLUSSVORRICHTUNG FÜR WEGWERFHÖSCHEN MIT ELASTISCHEN TAILLENBANDEN
SYSTEME DE FERMETURE POUR CULOTTE A ENFILER JETABLE A CEINTURE ELASTIQUE

(30) Priority: 29.04.1994 US 236594; 04.01.1995 US 368200
(43) Date of publication of application: 12.02.1997
(73) Proprietor: THE PROCTER & GAMBLE COMPANY, Cincinnati, Ohio 45202 (US)
(72) Inventor: RONN, Karl Patrick, Cinninnati, OH 45243 (US); ROE, Donald Carroll, West Chester, OH 45069 (US)
(74) Representative: Borbach, Markus
(86) International application number: PCT/US1995/005354
(87) International publication number: WO 1995/029657

(56) References cited:
- EP-A- 0 206 208
- EP-A- 0 570 980
- WO-A-91/08725
- FR-A- 2 331 975
- GB-A- 1 520 740
- GB-A- 2 244 422
- US-A- 4 988 346
- US-A- 5 246 433
- US-A- 5 304 162

## Description

### FIELD OF THE INVENTION

This invention relates to pull-on, stretchable waistband, disposable pants and more specifically to disposable pants having a stretchable waistband and a closure system, such as fasteners, to ensure that the pants snugly fit the wearer and to further ensure that the pants do not gap or leak at the waist

### BACKGROUND OF THE INVENTION

Disposable undergarments have been designed to address the needs of toddlers and young children for pull-up training pants. Additionally, larger versions of such disposable undergarments may be fabricated for use by older individuals with incontinence. Present incontinence products for adults typically are an absorbent member or pad designed to be worn in the crotch and held in position by means of straps or fasteners extending from the back of the article around the wearer to the front panel and attached at points near the end of the absorbent pad. The product essentially is a diaper which relies exclusively on the attachment straps for application and retention.

Whether they be toddlers or adults, the wearers have varying waistlines; as a result, a significant number of different sizes of garments to accommodate all individuals would be required. It is desirable to offer limited numbers of sizes in the product line from a production and inventory viewpoint while at the same time being able to accommodate a broad spectrum of waist sizes for the consumer.

For those wearers whose waist is slightly smaller than the optimum waistline for the pant, the pants could have a tendency to slip off the wearer or gap at the waist leading to leaks, particularly when the garment is loaded with urine or other body excrement. The pant garment may have sufficient extendibility in its elastized sections to permit the easy application of the pants to the wearer. But whenever the wearer's hips are insufficiently defined relative to the waist, as with toddlers and small children, this easy application to the wearer also may mean there could be insufficient forces generated between the hips of the wearer and the absorbent pant garment to retain the garment in the usual wearing position without gaps that can lead to leaks.

With regard to adult garments of a similar type for use by incontinent individuals, the definition between the waist and hips of an older adult in some cases is insufficient to provide good retention of the pant garment when using only the elastic forces within the side panels and the elastic forces of the waistband of the disposable, absorbent pant.

Incontinent adults often resist the use of diapers as demeaning and may be more comfortable using a pant- type garment. In addition to absorbency and disposability in products for incontinent adults, the requirements for such a pant-type structure include also comfort, reliability and dignity, additionally, toddlers or children engaged in toilet training need such a pant-type structure for practice to positively reinforce their training.

### SUMMARY OF THE INVENTION

It is an object of the invention to anchor pull-on pants of the disposable, absorbent type to the wearer while at the same time to retain the advantages of the pull-on type garment.

It is another object of the invention to increase and supplement the elastic forces necessary to anchor a pull-on stretchable disposable pant product on the wearer.

A still further object of the invention is to effectively adjust or reduce the waist size of a pull-on, elasticized, disposable, absorbent pant whenever the waist dimensions of the pant are slightly too large for the wearer's hips and waist, or when the pant gaps at the waist.

GB 2 244 422 describes a brief type disposable diaper.

An absorbent, disposable pant-type product as in U.S. Patent 5,246,433, issued to Hasse, et al., is known and used as a training pant for toddlers and young children and comprises the preamble of claim 1. As part of the body function control training for toddlers, it is desirable to provide a garment of the same general type as the youngster will be expected to wear after being fully trained. Part of this training includes learning how to and when to remove the garment in order to use the toilet. This requires that the toddlers or young children be provided with a garment which will slip on and off over the hips to permit ease of removal. Further, should the garment not be soiled, the garment then might be reused by pulling the garment up over the hips for continued wear

Training pant-type garments are designed with stretchable or elasticized waistbands and side panels in order to permit the extension of the garment to a larger size whenever the garment is pulled over the hips of the wearer. The forces required to stretch the garment adequately must not be excessively large or the individual may not be able to adequately remove the garment at the appropriate time and thereby losing the positive reinforcement of success and to replace the garment in the proper position. Lower elastic forced may be desirable in order to maximize the ease of application, removal or general wearing comfort. Therefore, the extension forces must be controlled and in some cases may be insufficient to retain the garment in the normal wearing position.

Accordingly, it is desirable to supplement the elastic waistband and side panel retention forces by addition of fasteners which will ensure that adequate forces are exerted between the wearer and the garment to retain the garment in the normal wearing position. The fasteners typically are formed as a strap or strip of material which is anchored either the back or front panel of the pant-type garment. The fasteners are anchored firmly to the garment and may be held in a retracted or stored position, such as folded, ready for use. Alternatively, the fasteners may be pre-fastened in the front of the garment and need only to be released prior to the garment being put on the wearer. After the garment has been placed on the wearer, the fastener may be pulled across the side panels and engaged with the front panel in a designated engagement or landing zone. As a further alternative, the fasteners may be extended to overlap and attach to each other where one fastener acts as a landing zone for the other. The fastener may be pulled sufficiently tight and the side panels of the garment gathered manually, if necessary, to ensure that the waist size of the pant is adequately reduced to prevent the pant from slipping inadvertently over the hips of the wearer and to reduce the likelihood of gapping at the waist that can lead to leaks.

The fastener may be one of several different types. The fastener preferably may be either an adhesive or a mechanical fastener. Further, the fastener may be a non-elastic strip or strap, or may be an extensible elastic strip.

For adhesive fasteners, the adhesive may be chosen from known adhesives to provide either a highly adherent force for firm attachment or a moderate tack adhesive for easier release of the adhesive from the material of the landing zone, thereby permitting re-use of the garment and the re-adherence of the fastener to the landing zone.

The fastener alternatively may be a mechanical fastener such as a hook/loop fastener similar to VELCRO brand fasteners, snaps, or a hook/perforation type fastener.

Either the mechanical fastener or the adhesive fastener may be retained in a Z-fold, and then unfolded to extend the fastener across the stretch panel at the sides of the garment for engagement with the front panel. For adhesive fasteners, the adhesive surface may be engaged with and protected by face-to-face engagement with a release surface on the Z-fold fastener. Thus, the adhesive may be removed from the release surface but yet retain adequate adhesion to the outer layer of the garment whenever engaged with the landing zone.

To retain the mechanical fasteners in the Z-fold unused position, a small spot or region of adhesive is applied between adjacent segments of the strip of the mechanical fastener. This application of adhesive will serve to retain the fastener in its folded and unused position until such time as the fastener is to be unfolded, extended and engaged with the front panel of the absorbent garment.

While the preceding summary has primarily focused upon and is particularly relevant to training pants for toddlers, the same principles and the same embodiments may be used on larger size garments for those older individuals with incontinence. Regardless of the intended wearer, the pants may incorporate an elastic or stretchable member disposed to partially encircle the waist of the wearer. Whenever the fasteners are extended and pulled toward the front panel of the garment, the elastic member partially surrounding the waist thus will be extended and will offer resistance to the pulling of the fastener toward its landing zone. As the elastic or extendable portion of the garment is stretched by pulling the fastener toward its landing zone, the forces resisting such pulling will increase and thus will provide the forces necessary to more firmly anchor the garment on the wearer The amount of extension may be varied to control the forces generated by the use of the fasteners. Particularly in the adult incontinence garment, a substantial anchoring force exerted by the fasteners around the waist of the wearer will give the wearer a tactile reassurance that the garment is in its proper place and additional reassurance that the garment will be more firmly retained in the appropriate wearing position. Such confidence is very important to maintaining the dignity of an individual with incontinence.

A better understanding of the invention may be had from the accompanying drawings and the detailed description to follow.

### BRIEF DESCRIPTION OF THE DRAWINGS

FIG. 1 is a right rear elevated perspective view of an absorbent, disposable pull-on training pant incorporating the invention.

FIG. 2 is an illustration of the chassis of a pull-on, absorbent, garment prior to the joining of the elasticized ears that form a side panel and the figure details placement of the fasteners relative to the chassis.

FIG. 3 illustrates the pull-on pants in a partial top view with the fastener in a folded condition prior to use.

FIG. 4 illustrates a fastener in extended condition prior to engagement with the front panel.

FIG. 5 illustrates a fastener extended and engaged with the landing zone of the garment.

FIG. 6 illustrates the disposable, absorbent pant garment shown in partial segments with a column/hook fastener and a perforated outer layer landing zone of the pants for engagement with the column/hook fastener.

FIG. 7 illustrates in segmented form a disposable pull-on absorbent garment with a fastener incorporating hook/loop attachment portions.

FIG. 8 illustrates the absorbent pant in partial segments with a pressure engageable snap.

FIG. 9 is an elevated front view of the absorbent pant with a fastener disposed on straps to engage and attach to each other on the front of the pant.

### DETAILED DESCRIPTION OF THE INVENTION

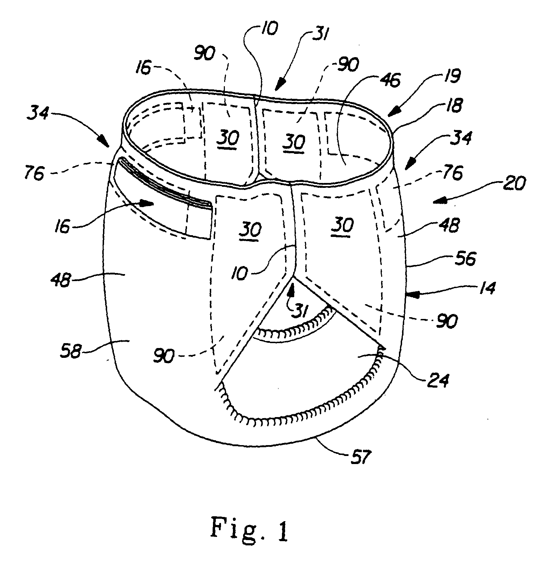

Referring initially to FIG. 1, a pull-on, disposable, absorbent pant-type garment 20 is illustrated having a front panel 56, a crotch panel 57 and a back panel 58. This type garment 20 is more fully and completely described in U.S. Patent 5,246,433 issued to Hasse, et al., September 21, 1993, and assigned to The Procter and Gamble Company, Cincinnati, Ohio. U.S. Patent 5,246,433.

This garment may be made in a plurality of different sizes and may be sold for uses including body function control training for toddlers as well as for use by incontinent adults.

Elasticized flaps 30 extend respectively from front panel 56 and back panel 58. Any convenient and appropriate technique such as gluing, ultrasonic bonding, heat welding or adhesive attachment, may be utilized to form seam 10 to complete the pant-type garment 20 as illustrated. Incorporated into the waistband region 34 near the waist opening 19 are elastic waistband members 76. The elastic members 76 preferably are confined between the inner layer 46 and the outer layer 48 of the garment 20. The inner layer and outer layer 46, 48, respectively, form the chassis 14 of the pant 20. Similarly, elastic panels 90 may be disposed between the inner layer 46 and outer layer 48 in the regions of the elasticized flaps 30 to provide the elastic stretchability aspect. Alternatively, the elastic panels may be exposed and not be contained between layers 46, 48.

Fastener 16 is disposed, preferably, on the back panel 58 of pant 20 and overlying at least a portion of the surface of the pant 20 in the waistband region 34 The waistband region 34 is that region of the outer layer 48 overlying elastic members 76, and proximate to the edge 18 of the pant 20 at the waist opening 19.

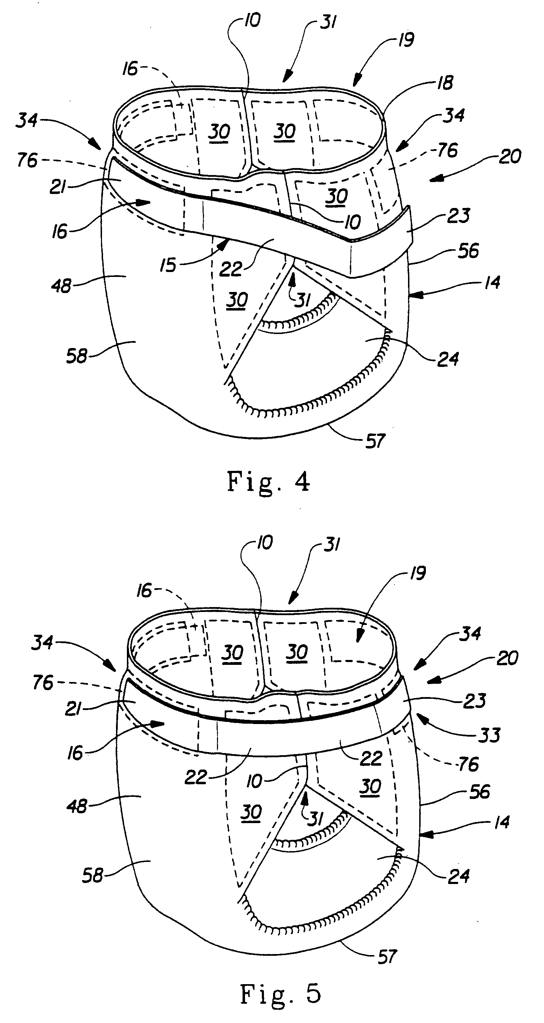

Fastener 16, comprising a strap 15 as later shown in FIG 4, may be disposed on the front panel 56 of the pant 20, if desired, but it is generally preferred that the fastener 16 be anchored to the back panel 48 so that the fastener 16 may be extended forward and readily .ttached to the front panel 56 by the wearer or the person giving assistance to the wearer.

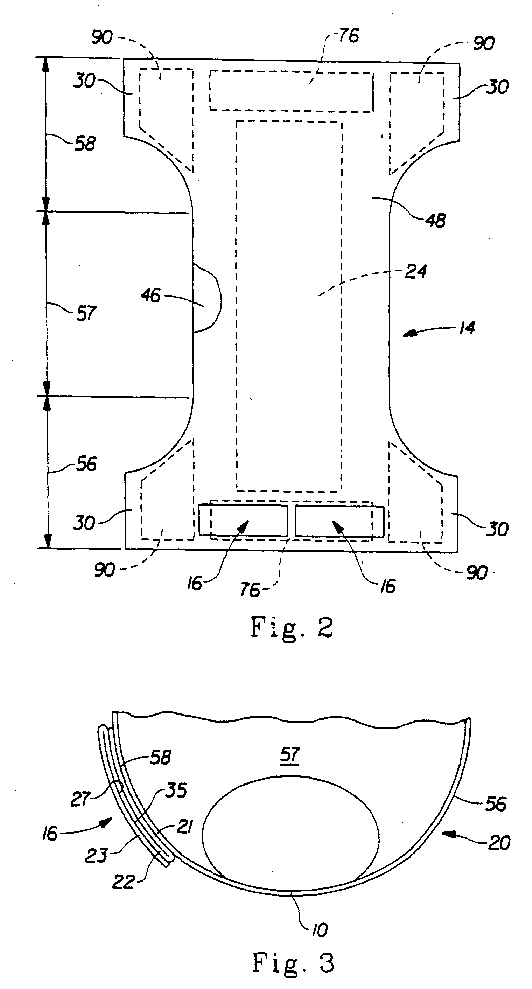

Referring now to FIG. 2, the construction of the chassis 14 is more readily apparent. Chassis 14 comprises liquid impervious outer layer 48, absorbent pad 24, elastic panels 90 and elastic members 76. The elastic panels 90 and elastic members 76 together with absorbent pad 24 may be trapped between the impervious outer layer 48 and a pervious inner layer 46, as is more thoroughly described in U.S. Patent 5,246,433. The chassis 14 supports fasteners 16 which are disposed generally overlying one of the elastic members 76. Elastic panels 90 may be exposed and be joined at their edges to the flap 30 or to the edges of panels 90. The essential aspect of elastic panels, such as 90, is to provide expandability to the waist of the pant 20.

The chassis 14 then may be folded and the elasticized ears 30 may be joined together using adhesive, glue, heat or ultrasonic bonding forming side panels 31. Other conventional joining techniques may be equally advantageous.

Reference is now made to FIG. 3, which illustrates pant 20 of the nature described in FIGS. 1 and 2 showing the attachment of one of the fasteners 16. Fastener 16 is preferably comprised of three segments, 21, 22 and 23. Segment 21 is anchored to the back panel 58 of the pant 20 by a high-strength adhesive, thermal bonding or ultrasonic bonding. The technique of adhesive attachment lends itself to manufacturability, serviceability and durability during use.

Segment 22 is folded back over segment 21 and may be retained in that position by a small region of adhesive 25. The adhesive 25 may be a low-tack adhesive merely sufficient to hold segment 22 in the folded-back position overlying segment 21.

Segment 23 of fastener 16 may be adhesively retained either by adhesive 25 on the surface 27 of segment 23 or a small quantity of adhesive 25 restricted to a small region of surface 27 merely used to tack and hold the segment 23 to segment 22.

In FIG. 3, with the segment 23 and particularly surface 27 coated with an adhesive, the fastener 16 may be unfolded and extended past seam 10 to a position where the surface 27 of segment 23, carrying an adhesive layer 35, may be then engaged with front panel 56, by pulling segment 23 and its adhesive coating 35 from segment 22. The fastener 16 may be pulled to extend the elastic member 76, as illustrated in FIGS. 1 and 2, creating forces to effectively reduce the waistband 34 circumference of the pant 20. The waistband 34 circumference is effectively reduced since the side panels 31 and elastic ears 30 are gathered or folded when the fastener 16 is pulled and engaged with front panel 36.

FIG. 4 illustrates the fastener 16 with segment 21 firmly attached to the back panel 58 of pant 20, intermediate segment 22 extended, and segment 23 illustrated as being positioned for engagement with the front panel 56 of pant 20.

FIG. 5 is a further illustration of fastener 16 in its fully extended and engaged condition whereby segment 22 extends across the elasticized ears 30 and side panels 31, and segment 23 adhesively engages the outer layer 48 in the general region of the elastic member 76. Accordingly, the region of engagement forms a landing zone 33 for segment 23 of fastener 16 by pulling the fastener 16 snug and engaging the outer layer 48 of pant 20 at landing zone 33 in the region of elastic member 76 and releasing the fastener 16; the restorative action of member 76 will have the effect to reduce the circumference of the waist opening 19 of the pant 20 to more closely conform to the waist of the wearer.

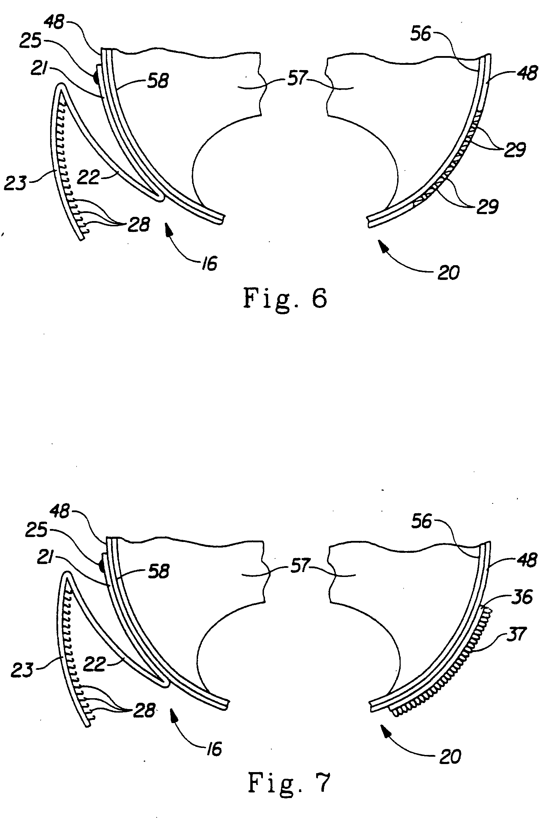

Referring now to FIG. 6, the disposable pant 20 is illustrated in segmented form with an alternative embodiment of fastener 16. Segments 21 and 22 are as previously described, with respect to FIGS. 3, 4 and 5, while segment 23 carries on one surface 27 thereof a plurality of hook members 28. The hook members 28 may be deposited and formed onto segment 23 in accord with the teachings with U.S. Patents 5,116,563 entitled PROCESS FOR PRODUCING A MECHANICAL FASTENER, issued to Dennis A. Thomas, et al., on May 26, 1992; U.S. Patent 5,180,534 entitled PROCESS OF MANUFACTURING A REFASTENABLE MECHANICAL FASTENING SYSTEM, issued to Thomas, et al. on January 19, 1993; and U.S. Patent 5,230,851 entitled PROCESS OF MANUFACTURING A REFASTENABLE FASTENING SYSTEM, issued to Thomas on July 27, 1993.

The hook members 28 may engage with and penetrate the perforations 29 which are formed through at least a portion of the front panel 56 forming a landing zone 33 of pant 20 and specifically through the outer layer 48. As fastener 16 is released after engaging hooks 28 into perforations 29, the contracting of elastic forces of elastic member 76, as previously illustrated in FIGS. 1, 2, will attempt to pull hooks 28 from perforations 29. The hooks 28 having been engaged with the outer layer 48 will retain hooks 28 and segment 23 in the position generally overlying elastic member 76, as previously described with respect to FIGS. 1 and 2. One skilled in the art will recognize that the mating portions of the fastener, hooks 28 and perforation 29, may be reversed in location.

Another alternative embodiment of the fastener is illustrated and described with respect to FIG. 7, to which we refer at this point.

Fastener 16 in FIG. 7 is substantially identical to the fastener 16 in FIG. 6. Segment 23 of the fastener 16 comprises a plurality of hook members 28 on one surface 27 thereof.

The front panel 56 of the pant 20, specifically outer layer 48, supports a backing sheet 36 and a loop pile 37. (However, the outer layer 48 of the pant 20 may be formed form a material that comprises the loop pile 37.) As used herein, the term "loop pile" refers to any loop type fastening element capable of engaging a complementary hook component. Some non-limiting examples of loops suitable for use with the present invention include loops formed by knitting loops into a backing, by pleating or corrugating fibers; or those described in allowed application Serial Number 07/703,441, filed by David Goulait on May 20, 1991, and U.S. Patent 5,032,122, entitled LOOP FASTENING MATERIAL FOR FASTENING DEVICE AND METHOD OF MAKING SAME, issued to Noel et al. on July 16, 1991. (It should also be noted that the loop pile 37 can be replaced with hook members 28, such that the fastener 16 is closed by engaging the hook members 28 with complementary hooks positioned where the loop pile 37 is located.)

Backing sheet 36 is preferably glued or adhesively attached to the outer layer 48 in the desired location, which is substantially the same location as that described for perforations 29, and backing sheet 36 supports the loop pile 37. The loop pile 37 then is engaged by the hooks 28 when the fastener 16 is extended and pulled forward to tighten the waist of pant 20 and pressed into engagement with the loop pile 37. After the pant 20 is applied to the wearer and fastener 16 extended to engage hooks 28 with loop pile 37 and thereafter it is desired to remove the pant 20, the hooks 28 may be disengaged from the loop pile 37 by lifting and pulling segment 23, which in turn will relieve the forces exerted by the fastener 16 onto the pant 20, and will permit the pant 20 to be slid over the hips of the wearer and removed. Should the pant 20 remain unsoiled, it may again be put onto the wearer, the fastener 16 again extended, and hooks 28 engaged with the loop pile 37 to increase the forces exerted onto the wearer necessary to hold the pant 20 in place. The fastener may be either a stretchable or non-elastic strap.

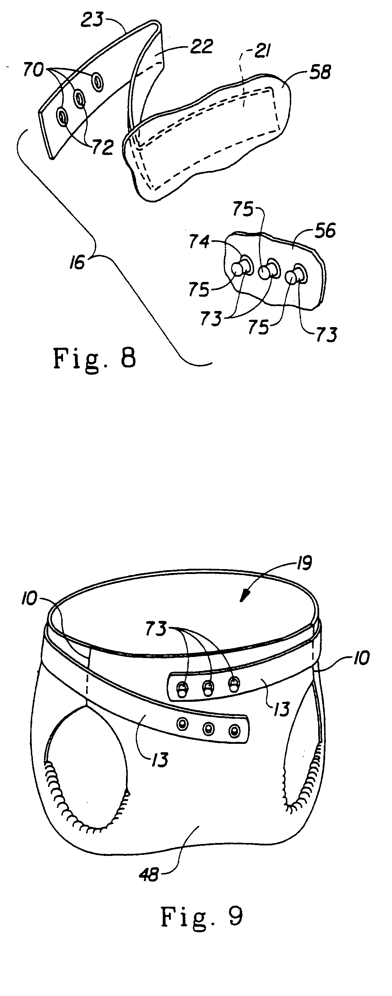

A still further embodiment of fastener 16 which may be incorporated with the pant 20 is illustrated in fragmentary representation in FIG. 8.

Fragments of back panel 58 and front panel 56 of the pant 20, as illustrated in FIG. 1, are shown with the segment 21 anchored to back panel 58. Segment 22 of the fastener and segment 23 are partially extended. Segment 23 has formed thereon a part of a snap fastener which is comprised of a post 73 and grommet 70 fastener. In the illustrated embodiment of FIG. 8, the grommets 70 may be attached by adhesive, glue, or by heat bonding to segment 23. The grommets 70 preferably would be a plastic ring having a central opening 72. The grommet 70 may engage a second element of the fastener 16 which is presented in the form of a post 73 supported on a reinforcing disk 74. Post 73 is provided with a bulbous head 75 of controlled diameter. The head 75 of post 73 may be positioned into central opening 72 of grommet 70, and then grommet 70 and head 75 may be forced toward each other until head 75 is forced through the central opening 72.

The yielding nature of plastic is advantageously used to form the grommet 70, the post 73, head 75 and support disk 74. To remove the fastener 16 from engagement with front panel 56, segment 23 of fastener 16 may be pulled generally outward to remove head 75 from the central opening 72 of grommet 70, thereby disconnecting the grommet 70 from the front panel 56 of a disposable pant 20.

In order to accommodate various waist sizes of wearers, segment 23 may be provided with a plurality of grommets 70 while front panel 56 may be provided with a plurality of the post 73 and head 75 portions of the fastener 16. In addition to being able to engage any one of the grommets 70 with any one of the heads 75 and posts 73, the spacing between grommets 70 and the spacing between posts 73 may be varied thereby providing a wide variation of waistlines which may be accommodated by the fastener 16. Also, the grommets 70 and posts 73 may be formed on separate fastener straps 13 and thus engageable. The fastener straps 13 will effectively reduce the waistband 34 size of the pant 20 without forming the grommets, or posts 73 on the front panel 56 of the pant 20.

By controlling the number of grommets 70 and the number of posts 73, the quantity of combinations of engaging locations may yield a significant number of variable waist sizes that may be accommodated by fastener 16. For example, for each fastener 16 having three grommets 70 and three posts 73, nine possible positional combinations are achievable. With two such fasteners 16, one on each side of the pant 20 as illustrated in FIG. 1, it is possible to create eighty-one different waist sizes over a relatively broad spectrum. Accordingly, a garment which may be slightly too large for a particular wearer may be adjusted to accommodate that wearer with waist size variations comparable to the large number of positions possible with either an adhesive fastener as illustrated and discussed with respect to FIG. 1, a hook 28/perforation 29 fastener 16 illustrated and discussed with reference to FIG. 6, or the hook 29/loop pile 37 fastener 16 of FIG. 7.

While the invention has been described generally with the segment 21 of fastener 16 anchored to the back panel 58 of the pant 20, it should be understood that the fastener 16 may be also anchored in a similar manner to the front panel 56. Front panel 56 attachment requires a landing zone 33 of perforations 29, loop pile 37, posts 73 or heads 75, or an open adhesively engageable area 33 for the adhesive fasteners to engage and adhere to the outer layer 48 of back panel 58. Additionally, it should be understood that the perforations 29 illustrated in FIG. 6, which extend through outer layer 48, may be formed into segment 23 of the fastener 16 and the hooks 28 may be positioned on outer layer 48 or on the other fastener 16 with equal efficacy. Similarly, hooks 28 and the backing sheet 36 with loop pile 37 may be interchanged from the position illustrated in FIG. 7 if desired or substituted for the posts 73 and grommets 70 of FIG. 9. The grommets 70 and the posts 73 and heads 75 similarly may be interchanged in position from that illustrated in FIG. 8 without losing efficacy.

In any of the embodiments of the fastener 16 illustrated in any of the drawings, the purpose is to allow the fastener 16 to be pulled snug and to effectively reduce the waist opening 19 size of a elasticized disposable pant 20, thereby permitting the pant 20 to be applied to the wearer by pulling the pant 20 up over the hips and into the normal wearing position and then supplementing the elastic forces of the elastic pant 20 to further ensure that the pant 20 remains in the normal wearing position until such time as the pant 20 is

## Claims

1. A pull-up absorbent disposable pant (20) comprising:
a chassis (14) including a liquid impervious outer layer (48) having an inner surface (46); said chassis (14) comprising a front panel (56), a back panel (58), each disposed at opposite ends of said chassis (14), and a crotch panel (57) intermediate said front and back panels (56, 58), and side panel flaps (30) extending laterally from said front and back panels (56, 58),
an absorbent core (24) disposed proximate said inner surface (46);
said side panel flaps (30) each having lateral edges and said front and back panels (56, 58) and said side panel flaps (30) having end edges (18);
said side panel flaps (30) of extending from said front panel (56) and said back panel (58) joined together forming side panels (31); elastic panels (90) associated with said side panels (31) providing elasticity to said side panels (31); elastic members (76) associated with said front panel (56) and said back panel (58) and disposed proximate to said end edges (18) forming an elastic waistband (34); fasteners (16) engageable and attachable to effectively reduce said waistband (34) size of said pant (20);
said lateral edges of said side panel flaps (30) being of similar length, said side panel flaps (30) extending from said front panel (56) and said back panel (58) being joined together (10) along the entire length of said lateral edges (10) of said side panel flaps (30), **characterized by** fasteners (16) each comprising an elongated layer disposed on and each anchored to one of said front and back panels (56, 58) by means of high-strength adhesive, thermal bonding of ultrasonic bonding and overlaying the elastic member (76) associated with said front or back panel (56,58) such that pulling on the fastener (16) extends the elastic member (76) and extendible to the other of said back and front panels (58, 56), across said side panels (31), said fasteners (16) preferably being disposed proximate to one of said end edges (18) of said chassis (14).

2. The pant (20) of claims 1 wherein said fastener (16) is adhesively attachabie to said other of said back or front panel (58, 56).

3. The pant (20) of claims 1 or 2 wherein said fastener (16) is folded upon itself and adhesively retained in said folded condition prior to use.

4. The pant (20) claims 1 or 3 wherein said fastener comprises a plurality of hooks (28) and a plurality of loops (37) a plurality of hooks (28) engageable with another plurality of hooks (28) or at least one grommet (70) and at least one post (73), whereby said grommet (70) may be forced over said post (73).

5. The pant (20) of claim 4 wherein said at least one post (73) is attached to said pant (20) proximate to and adjacent to said end edge (18) of one of said back or front panels (58, 56).

6. The pant (20) of claims 1 or 3 wherein said fastener (16) further comprises a plurality of hooks (28) disposed on one of said layers and a plurality of apertures (29) defined by another one of said layers for engagement and retention by said hooks (28).

7. The pant (20) of claim 6 wherein said plurality of apertures (29) is disposed proximate one said end edge of said outer layer (48).

8. The pant (20) of claims 1 or 3 wherein said fastening means comprises a plurality of grommets (70) and a plurality of posts (73), one of said pluralities of grommets (70) and posts (73) disposed on a strap (13) disposed on either said front or said back panel (56, 58) and said other of said pluralities of said grommets (70) and posts (73) disposed on the other of said front or back panel (56, 58), grommets (70) engageable with and detachably retainable by said posts (73) whereby said strap (13) may be engaged with and attached to said other of said front and back panels (56, 58); said grommets (70) disposed regularly spaced apart from the others of said grommets (70) by a uniform distance and each of said posts (73) of said plurality of posts (73) are regularly spaced apart from the others of said posts (73) by a uniform distance, said distance between said grommets (70) and said distance between said posts (73) being unequal.

## Patentansprüche

1. Eine absorbierende Wegwerfhose (20) zum Hochziehen, welche umfasst:
eine Grundstruktur (14), welche eine flüssigkeitsundurchlässige äußere Schichte (48) inkludiert, mit einer inneren Oberfläche (46); wobei die genannte Grundstruktur (14) ein vorderes Feld (56), ein hinteres Feld (58), wobei jedes an gegenüberliegenden Enden der genannten Grundstruktur (14) angeordnet ist, und
ein Schrittfeld (57) zwischenliegend zwischen dem genannten vorderen und dem genannten hinteren Feld (56, 58) und Seitenfeldlappen (30) umfasst, welche sich von den genannten vorderen und hinteren Feldern (56, 58) quer erstrecken;
einen absorbierenden Kern (24), welcher nächstgelegen der genannten inneren Oberfläche (46) angeordnet ist;
wobei die genannten Seitenfeldlappen (30) jeweils Seitenränder aufweisen und die genannten vorderen und hinteren Felder (56, 58) und die genannten Seitenfeldlappen (30) Stirnränder (18) aufweisen; wobei die genannten Seitenfeldlappen (30), die sich vom genannten vorderen Feld (56) und vom genannten hinteren Feld (58) erstrecken, Seitenfelder (31) bildend miteinander verbunden sind;
elastische Felder (90), welche mit den genannten Seitenfeldern (31) verbunden sind, wobei sie den genannten Seitenfeldern (31) Elastizität verleihen;
elastische Bauteile (76), welche mit dem genannten vorderen Feld (56) und dem genannten hinteren Feld (58) verbunden sind und nächstgelegen den genannten Stirnrändern (18), welche ein elastisches Taillenband (34) bilden, angeordnet sind;
Befestigungsmittel (16), welche in Eingriff gelangen können und fixierbar sind, um die genannte Taillenband-(34)-Größe der genannten Unterhose (20) wirksam zu reduzieren;
wobei die genannten Seitenränder der genannten Seitenfeldlappen (30) von ähnlicher Länge sind, wobei die genannten Seitenfeldlappen (30), die sich vom genannten vorderen Feld (56) und vom genannten hinteren Feld (58) erstrecken, entlang der gesamten Länge der genannten Seitenränder (10) der genannten Seitenfeldlappen (30) miteinander verbunden sind, **dadurch gekennzeichnet, dass** die Befestigungsmittel (16) jeweils eine längliche Schichte umfassen, welche an einem der genannten vorderen und hinteren Felder (56, 58) angeordnet und jeweils mittels hochfestem Klebstoff, thermischer Bindung oder Ultraschallbindung daran befestigt ist und den elastischen Bauteil (76) bedeckt, welcher mit dem genannten vorderen Feld (56) oder dem genannten hinteren Feld (58) derart verbunden ist, dass ein Ziehen am Befestigungsmittel (16) den elastischen Bauteil (76) verlängert und zum anderen der genannten hinteren und vorderen Felder (58, 56) über die genannten Seitenfelder (31) verlängerbar ist, wobei die genannten Befestigungsmittel (16) nächstgelegen zu einem der genannten Stirnränder (18) der genannten Grundstruktur ( 14) angeordnet sind.

2. Die Hose (20) nach Anspruch 1, bei welcher das genannte Befestigungsmittel (16) an dem genannten anderen des genannten vorderen oder hinteren Feldes (58, 56) klebend fixierbar ist.

3. Die Hose (20) nach Anspruch 1 oder 2, bei welcher das genannte Befestigungsmittel (16) um sich selbst gefaltet ist und vor dem Gebrauch in dem genannten gefalteten Zustand klebend festgehalten ist.

4. Die Hose (20) nach Anspruch 1 oder 3, bei welcher das genannte Befestigungsmittel eine Mehrzahl von Haken (28) und eine Mehrzahl von Schlaufen (37), wobei eine Mehrzahl von Haken (28) mit einer anderen Mehrzahl von Haken (28) in Eingriff gelangen kann, oder mindestens eine Öse (70) und mindestens einen Stift (73), wobei die genannte Öse (70) über den genannten Stift (73) gezwängt werden kann, umfaßt.

5. Die Hose (20) nach Anspruch 4, bei welcher der genannte mindestens eine Stift (73) an der genannten Hose (20) nahe dem und anliegend an den genannten Stirnrand (18) von einem der genannten hinteren oder vorderen Felder (58, 56) fixiert ist.

6. Die Hose (20) nach Anspruch 1 oder 3, bei welcher das genannte Befestigungsmittel (16) weiters eine Mehrzahl von Haken (28), welche an einer der genannten Schichten angeordnet sind, und eine Mehrzahl von Öffnungen (29), welche durch eine andere der genannten Schichten definiert sind, zum Ineinandergreifen und Zurückhalten durch die genannten Haken (28) umfaßt.

7. Die Hose (20) nach Anspruch 6, bei welcher die genannte Mehrzahl von Öffnungen (29) nahe einem genannten Stirnrand der genannten äußeren Schichte (48) angeordnet ist.

8. Die Hose (20) nach Anspruch 1 oder 3, bei welcher das genannte Befestigungsmittel eine Mehrzahl von Ösen (70) und eine Mehrzahl von Stiften (73) umfaßt, wobei eine der genannten Mehrzahl von Ösen (70) und Mehrzahl von Stiften (73) an einem Bügel (19) angeordnet ist, welcher an entweder dem genannten vorderen Feld (56) oder dem genannten hinteren Feld (58) angeordnet ist und bei welcher die genannte andere der genannten Mehrzahlen von genannten Ösen (70) und Stiften (73) an dem anderen des genannten vorderen Feldes (56) oder hinteren Feldes (58) angeordnet ist, wodurch die genannten Ösen (70) mit den genannten Stiften (73) in Eingriff bringbar sind und durch dieselben lösbar festgehalten werden können, wodurch der genannte Bügel ( 19) mit dem genannten anderen von den genannten vorderen und hinteren Feldern (56, 58) in Eingriff gelangen und daran fixiert werden kann;
wobei die genannten Ösen (70) in einer einheitlichen Distanz von den anderen der genannten Ösen (70) regelmäßig beabstandet angeordnet sind, und jeder der genannten Stifte (73) der genannten Mehrzahl von Stiften (73) von den anderen der genannten Stifte (73) in einer einheitlichen Distanz regelmäßig beabstandet ist,
wobei die genannte Distanz zwischen den genannten Ösen (70) und die genannte Distanz zwischen den genannten Stiften (73) ungleich sind.

## Revendications

1. Culotte absorbante jetable à enfiler (20) comprenant :
un châssis (14) comprenant une couche extérieure (48) imperméable aux liquides présentant une surface intérieure (46) ; ledit châssis (14) comprenant un panneau avant (56), un panneau arrière (58), chacun placé à des extrémités opposées dudit châssis (14), et un panneau d'entrejambe (57) entre lesdits panneaux avant et arrière (56, 58) et des rabats de panneau latéraux (30) s'étendant latéralement à partir desdits panneaux avant et arrière (56, 58),
une âme absorbante (24) placée à proximité de ladite surface intérieure (46) ;
lesdits rabats de panneau latéraux (30) présentant chacun des bords latéraux et lesdits panneaux avant et arrière (56, 58) et lesdits rabats de panneau latéraux (30) présentant des bords d'extrémité (18) ;
lesdits rabats de panneau latéraux (30) s'étendant à partir dudit panneau avant (56) et dudit panneau arrière (58) réunis ensemble formant des panneaux latéraux (31 ) ;
des panneaux élastiques (90) associés auxdits panneaux latéraux (31) fournissant une élasticité auxdits panneaux latéraux (31) ; des éléments élastiques (76) associés audit panneau avant (56) et audit panneau arrière (58) et placés à proximité desdits bords d'extrémité (18) formant une bande de ceinture élastique (34) ; des dispositifs de fixation (16) pouvant venir en prise et être fixés pour réduire efficacement la taille de ladite bande de ceinture (34) de ladite culotte (20) ;
lesdits bords latéraux desdits rabats de panneau latéraux (30) étant de longueur semblable, lesdits rabats de panneau latéraux (30) s'étendant à partir dudit panneau avant (56) et dudit panneau arrière (58) étant réunis ensemble suivant l'entière longueur desdits bords latéraux (10) desdits rabats de panneau latéraux (30), des dispositifs de fixation (16) comprenant chacun une couche allongée placée sur un desdits panneaux avant et arrière (56, 58) et ancré chacun à un de ceux-ci par des moyens de liaison à haute résistance adhésive ou thermique, ou de liaison aux ultrasons et recouvrant l'élément élastique (76) associé auxdits panneaux avant et arrière (56, 58) de manière que l'allongement sur le dispositif de fixation (16) étire l'élément élastique (76), et extensible jusqu'à l'autre desdits panneaux avant et arrière (56, 58) à travers lesdits panneaux latéraux (31), lesdits dispositifs de fixation (16) étant placés à proximité de l'un desdits bords d'extrémité (18) dudit châssis (14).

2. Culotte (20) selon la revendication 1, dans laquelle ledit dispositif de fixation (16) peut être fixé de manière adhésive audit autre panneau desdits panneaux avant et arrière (56, 58).

3. Culotte (20) selon la revendication 1 ou la revendication 2, dans laquelle ledit dispositif de fixation (16) est plié sur lui-même et retenu de manière adhésive dans ledit état plié avant l'utilisation.

4. Culotte (20) selon la revendication 1 ou la revendication 3, dans laquelle ledit dispositif de fixation comprend une pluralité de crochets (28) et une pluralité de boucles (37), une pluralité de crochets (28) pouvant venir en prise avec l'autre pluralité de crochets (28) ou au moins un oeillet (70) et au moins une pièce saillante (73), de sorte que ledit oeillet (70) peut être poussé sur ladite pièce saillante (73).

5. Culotte (20) selon la revendication 4, dans laquelle au moins une pièce saillante (73) est fixée à ladite culotte (20) à proximité dudit bord d'extrémité (18) de l'un desdits panneaux avant et arrière (56, 58), et de manière adjacente à celui-ci.

6. Culotte (20) selon la revendication 1 ou la revendication 3, dans laquelle ledit dispositif de fixation (16) comprend, en outre, une pluralité de crochets (28) placés sur une desdites couches, et une pluralité d'ouvertures (29) définies par l'autre desdites couches en vue d'une prise et d'une retenue par lesdits crochets (28).

7. Culotte (20) selon la revendication 6, dans laquelle ladite pluralité d'ouvertures (29) est placée à proximité d'un dit bord d'extrémité de ladite couche extérieure (48).

8. Culotte (20) selon la revendication 1 ou la revendication 3, dans laquelle ledit moyen de fixation comprend une pluralité d'oeillets (70) et une pluralité de pièces saillantes (73), une desdites pluralités d'oeillets (70) et de pièces saillantes (73) placée sur une sangle (13) placée sur l'un ou l'autre desdits panneaux avant et arrière (56, 58), et ladite autre desdites pluralités desdits oeillets (70) et pièces saillantes (73) placée sur l'autre desdits panneaux avant et arrière (56, 58), lesdits oeillets (70) pouvant venir en prise avec lesdites pièces saillantes (73) et être retenus de manière détachable par celles-ci, de sorte que ladite sangle (13) peut être prise avec ledit autre desdits panneaux avant et arrière (56, 58) et fixée à celui-ci ;
lesdits oeillets (70) sont placés régulièrement espacés des autres desdits oeillets (70) d'une distance uniforme, et chacune desdites pièces saillantes (73) de ladite pluralité de pièces saillantes (73) sont régulièrement espacés des autres desdites pièces saillantes (73) d'une distance uniforme, ladite distance entre lesdits oeillets (70) et ladite distance entre lesdites pièces saillantes (73) étant inégales.
